# EUROPEAN PATENT APPLICATION

(11) **EP 1 129 725 A2**
(43) Date of publication of application: **05.09.2001**
(21) Application number: 01103466.7
(22) Date of filing: 14.02.2001
(51) Int. Cl.: A61K 47/48

(54) **Oligonucleotide conjugates**

(30) Priority: 14.02.2000 DE 10006572
(71) Applicant: Deutsches Krebsforschungszentrum Stiftung des öffentlichen Rechts, 69120 Heidelberg (DE)
(72) Inventor: Eisenhut, Michael, 69118 Heidelberg (DE); Mier, Walter, 69115 Heidelberg (DE); Eritja, Ramon, Centro de Investigacion y Desarollo, 8034 Barcelona (ES); Haberkorn, Uwe, Schwetzingen (DE)
(74) Representative: Schüssler, Andrea, Dr.

(57) **Abstract**

The present invention relates to an oligonucleotide conjugate, comprising: (a) an oligonucleotide at least part of whose sequence is complementary to an intracellular nucleic acid sequence; and (b) a somatostatin analog. The present invention also relates to a medicament containing this oligonucleotide conjugate, preferably for treating tumors in which the somatostatin receptor (SSTR) is overexpressed.

## Description

The present invention relates to an oligonucleotide conjugate comprising: (a) an oligonucleotide at least part of whose sequence is complementary to an intracellular nucleic acid sequence; and (b) a somatostatin analog. The present invention also concerns a medicament containing this oligonucleotide conjugate, preferably for treating tissues in which the somatostatin receptor (SSTR) is overexpressed.

A large number of publications was published in recent years (Chrissey, Antisense Res. Devel. 1 (1991), 65), which dealt with the biological activity of synthetic antisense oligonucleotides (ODNs). The concept concealed behind the medical use of such ODNs is the intracellular hybridization with a corresponding sense sequence of the messenger RNA which codes for a certain protein. Following ODN mRNA duplex formation, RNase H is activated which hydrolytically destroys the mRNA. Other mechanisms inhibit the translation initiation of the splicing of pre-mRNA. These mechanisms inhibit the expression of a certain protein and can be repeated for an intact ODN. The effectivity of intracellular antisense ODNs should therefore be high. In addition to a number of promising experiments which were carried out using cell cultures, there were, however, serious retrogressive steps for the clinical application of first-generation antisense ODNs. Second-generation compounds comprised above all the phosphorothioates which distinguished themselves from the ODN derivatives tested by that time by a better *in vivo* stability over nucleases and an increased cell membrane permeability. The other modifications were carried out with the bases and the oligomeric backbone of the ODNs, which are not dealt with in more detail herein. The partially serious side-effects of the phosphorothioates comprised strong immune responses. The improvements which shall presently be obtained with the commercially favored phosphorothioates are, on the one hand, charge reduction by introducing uncharged oligo building blocks to reduce undesired side-effects and, on the other hand, sequence optimization to improve the affinity for the target mRNA.

It showed that the taking-up of oligonucleotides into the cell depends on the time, temperature, energy and concentration. Physico-chemical characteristics which influence particularly the pharmacokinetics of these compounds are lipophilia, the ionization degree and the molecular structure. In this connection, a limiting effect is in particular their polyanionic charge when passing through the positively charged cell membrane. The membrane transport and the cellular distribution vary strongly in the case of modified oligonucleotides as a function of the modified lipophilia and charge distribution (Crooke and Lebleu, Antisense Research and Applications (1993), CRC Press, Boca Raton, FL). Unmodified phosphodiester oligonucleotides are internalized by receptor-controlled endocytosis in the cell. These 34 to 90 kDa receptor proteins having obviously reduced transport capacity were identified using various methods (Loke et al., Proc. Natl. Acad. Sci U.S.A. 86 (1989), 3474; Yakubov et al., Proc. Natl. Acad. Sci. U.S.A. 86 (1989), 6454). However, their real functioning has not yet been deciphered. Methylphosphonates penetrate the cell by absorptive endocytosis (pinocytosis). Other modified oligonucleotides do not rely on a diffusion through the cell membrane either and are introduced by endocytosis without special receptors.

It is still unclear whether the contents of the endosome are readily released in the cytoplasm in the final analysis. The problem consists in the difficult detection of the oligonucleotides in the very close cell compartments of vacuoles, cytoplasm and cell nucleus by the current measuring methods (Milligan et al., J. Med. Chem. 36 (1993), 1923). Some authors report that only up to 10 % of the oligonucleotides migrate from the extracellular space into the cell interior (Edington, Bio/Technology 10 (1992), 993). Microinjections of natural and modified oligonucleotides having fluorescence labeling showed, however, under the fluorescence microscope a concentration of the sample in the cell nucleus within seconds. The correlation between the observations resulting from fluorescence microscopy and the biological activity of the compounds suggested the theory that the transport through the cell membrane and the release of the oligonucleotides from the vacuoles are counted among the most ineffective steps of the described transport mechanisms.

Many efforts have therefore been made to improve permeation. For example, phosphorothioates have an increased antisense activity in the presence of cationic lipids, however, they have none in the presence of anionic lipids (Colige et al., Biochemistry 32 (1993), 7). Due to their increased lipophilia phosphorothioates have an improved passing capability through the cell membrane as compared to phosphodiesters. On the other hand, they may trigger the above-mentioned undesired side-effects which because of certain ODN sequences and modifications resulted in a massive cytokine release and subsequent immunostimulation (Krieg et al., Nature 374 (1995), 546; Stein et al., Gene 72 (1988), 333). Phosphorothioate oligodeoxynucleotides caused complement activation, clot formation and hypertension in monkeys (Galbraith et al., Antisense Res. Dev. 4 (1994), 201). In addition, these compounds have potential binding properties with respect to proteins, such as growth factors and receptors, resulting in numerous non-antisense effects (Mateucci and Wagner, Nature 384 (1996), 20; Stein and Cheng, Science 261 (1994), 1004; Hertl et al., J. Invest. Derm 104 (1995), 813; Maltese et al., Nucleic Acids Res. 23 (1995), 1146; Chavany et al., Molec. Pharmac. 48 (1995), 738).

Other strategies of improving the cell absorption pursued the induction of known transport mechanisms by linking certain groups. Examples are the conjugation of hydrophobic groups such as cholesterin, phospholipids or of charge-compensating poly(L-lysine) (PLL) chains (Crooke and Lebleu, Antisense Research and Applications (1993), CRC Press, Boca Raton, FL). Acridine conjugated at the 3' or 5' end of phosphodiester oligonucleotides showed along with an improved nuclease stability and a tighter hybridization by intercalation particularly favorable permeation effects with various cell types (Stein et al., Gene 72 (1988), 333). Another interesting approach for fixing the oligonucleotides on the cell surfaces is presently pursued by several study groups. The discovery of the Ca²⁺-dependent carbohydrate-recognizing domains (CRDs) in the receptors of macrophages prepared the ground for a cell-specific strategy. However, the receptors only detect structures having several sugar units. For mannose residues e.g. antisense oligonucleotides were joined with mannose residues via a high-molecular protein (Mosigny et al., Adv. Drug. Del. Rev. 14 (1994), 1) or mannose phosphoroamidites were introduced into ODNs in automated fashion (Wijsman et al., Recueil des Travaux Chimiques des Pays-Bas 115 (1996), 397). In summary, the therapeutic use of ODNs has only led to, on the whole, unsatisfactory results thus far, above all because of the very inefficient taking-up through the cell and the absence of a specific transport system for certain desired cells.

Thus, it is the object of the present invention to provide antisense ODNs which do not have the drawbacks of the above described compounds used in therapy thus far, i.e. above all can be absorbed efficiently by the cell and can preferably be introduced into the desired target cells.

This technical problem is solved by the embodiments characterized in the claims.

The present invention served for developing ODN conjugates which can be taken up via somatostatin receptors (SSTRs). Somatostatin is a cyclic tetradecapeptide which is present in the hypothalamus, comprises important regulatory functions and has inhibitory effects as regards the secretion of growth hormones. In addition, SSTRs are overexpressed in various tumors, e.g. in small cell lung carcinomas, breast tumors, brain tumors, and many other (predominantly endocrine) tumors. Thus, the membrane-associated SSTRs represent a potential molecular target for the selective incorporation of desired active substances, in particular into tumors. The inventors then considered that it should be possible to link substances to be transported into cells to the natural substrate of SSTRs, i.e. to somatostatin, thus achieving selective cell uptake via SSTRs. However, the short biological half-life of natural somatostatin *in vivo* (less than 3 min.) is opposed thereto. However, some analogs of somatostatin have meanwhile been developed which should be suitable for the purpose according to the invention.

According to the invention conjugates are now provided comprising (a) an oligonucleotide (ODN) at least part of whose sequence is complementary to an intracellular nucleic acid sequence; and (b) a somatostatin analog. The conjugates according to the invention permit e.g. the well-calculated antisense therapy, above all of tumors in which SSTRs are overexpressed. In addition to the cancer therapy, the antisense oligonucleotides are adapted to treat viral diseases, such as HSV-1 (herpes simplex virus) diseases, to treat inflammatory processes (e.g. target RNA: NF-KB), to treat asthmatic diseases (e.g. target RNA: adenosine-Al receptor), to treat diseases of the central nervous system (e.g. target RNA: dopamine receptors), and to treat cardio-vascular diseases (e.g. target RNA: c-myc).

The oligonucleotides in consideration are both oligodeoxyribonucleotides and oligoribonucleotides, however, the latter preferably in stabilized form, e.g. 2-O-methyl-RNA. Yet due to their greater stability oligodeoxyribonucleotides are preferred.

Octreotide, octreotate, tyrosine-3-octreotide, tyrosine-3-octreotate, lanreotide and RC-160 (C.J. Andersen et al., Chem. Rev. 99, pages 2219-2234 (1999)) are preferred as somatostatin analogs. In addition, peptide mimetics such as PTR 3046 (Gilon et al., J. Med. Chem. 41, pages 919-929 (1998)) are suitable. Octreotide is a very effective analog of somastatin which is a cyclic octapeptide having an improved specificity and a half-life of about 90 min. (Bauer et al., Life Sci. 31 (1982), 1133-1140). It turned out recently that octreotate, the carboxylic acid derivative of octreotide, has even improved pharmacological properties and is thus an interesting alternative for octreotide (de Jong et al., Cancer Res. 58 (1998), 37-441). The expression "octreotide" used herein relates to a somatostatin analog having the structure described by Bauer et al., Life Sci. 31 (1982), 1133-1140 and the expression "octreotate" used herein refers to a somatostatin analog having the structure described by de Jong, Cancer Res. 58 (1998), 437-441, a carboxylic acid derivative of octreotide being concerned. These expressions also comprise modifications of the originally described molecules, e.g. modifications which relate to the exchange, deletion or addition of one or more amino acids and further modifications which are known to a person skilled in the art and which are common for peptides and do not impair, or do not impair substantially, the effectiveness of the molecule as somatostatin analog, e.g. tyrosine-3-octreotide and tyrosine-3-octreotate. Spacer-modified octreotide derivatives are also suitable (Smith-Jones et al., Nucl. Med. Biol. 24, pages 761-769 (1997)).

In a preferred embodiment of the present invention, the phosphodiester compounds of the oligonucleotide molecule according to the invention are partially or preferably fully replaced by phosphorothioate compounds. As a result, the oligonucleotide molecules have a greater half-life within the cell, since they are protected from enzymatic degradation. Methods of synthesizing such modified oligonucleotide molecules are known to the person skilled in the art and they comprise e.g. also the synthesis method described in Example 3 below.

Additional modifications may be introduced to further increase the stability of the oligonucleotide molecule according to the invention. Here, the protection of the 3' end of the ODN over the degradation by exonucleases using a propanediol group has to be mentioned by way of example. Further advantageous modifications are modifications of the internucleotide phosphodiester bridge, e.g. phosphorodithioate, methylphosphonate; modifications of the sugar residues, e.g. α-anomeric sugars, 2'-modified sugars, carbocyclic sugars; modifications of the bases, e.g. 5-propinyl-T, 7-desaza-7-propinyl-G (oligonucleotides and analogues, A practical approach, Editor: F. Eckstein, Oxford University Press 1991; Methods in Molecular Biology, Vol. 26: Protocols for oligonucleotide conjugates: Synthesis and Analytical techniques, Editor: S. Agrawal, Humana Press, 1994). Peptide-nucleic acid derivatives (e.g. PNA) are also usable.

In another preferred embodiment of the present invention, the oligonucleotide molecule is covalently linked with Tyr³-octreotate, e.g. at the 5' end or 3' end, and e.g. for the synthesis of this compound the steps may be taken as indicated in the below examples. This permits the radioactive labeling of the whole molecule so as to be able to pursue e.g. the efficiency of the binding to the SSTR and/or the uptake into the cell.

In a preferred embodiment phosphorothioate ODNs were used as a basis for the production of the conjugates according to the invention, which were covalently linked with Tyr³ octreotate. The peptide was synthesized via solid phase synthesis, oxidized to form the cyclic disulfide and then derivatized with an N-terminal maleimido unit. 5'-thiol-derivatized phosphorothioate ODNs which were directed against the proto-oncogene bcl-2 were attached by conjugation to this maleimido-modified peptide. It was found surprisingly that the above-mentioned prior art problems could be solved with these Tyr³ octreotide-linked antisense oligonucleotides, since it showed that the conjugates can still bind to SSTRs with high affinity, it also turning out that the terminal conjugation of the ODNs does not impair the binding to the target nucleic acid molecule.

Particularly preferred are oligonucleotide conjugates according to the invention in which the somatostatin analog, e.g. octreotide or octreotate is covalently linked with the 5' end of the oligonucleotide molecule, e.g. via a thioether linkage or linkage via acetal, alkine, amide, amine, carbamate, α-carbonyl thioether, disulfide, ester, ether, phosphoric acid ester, sulfonic acid ester or thiourea. The linkage with the 5' end of the oligonucleotide via a disulfide bridge is most preferred.

It is also preferred to link the somatostatin analog with a substituent of a base present in the oligonucleotide via a spacer. Preferably hydrocarbon groups, e.g. -(CH₂)₁₋₁₀-, most preferably methyl, ethyl, propyl groups, are suitable as spacers.

The nucleic acid sequence of the oligonucleotide molecule is designed in accordance with the desired target sequence, i.e. it must be substantially complementary thereto, so that hybridization can take place and thus the biological function of the intracellular target molecule can be inhibited. The intracellular nucleic acid sequence is preferably an mRNA, e.g. an mRNA coding for a prooncogene or oncogene, or viral RNA, e.g. of HIV. Oligonucleotide molecules whose nucleic acid sequences are complementary to the coding part of an mRNA are most preferred.

In order to ensure good uptake via the SSTRs, on the one hand, and permit a stable and specific attachment to the target nucleic acid sequence, on the other, the nucleic acid sequence of the oligonucleotide molecule according to the invention shall have a length between eight and fifty nucleotides, more preferably a length between twelve and twenty nucleotides, and most preferably about 15 nucleotides.

As discussed above already, the oligonucleotide molecules according to the invention are particularly suitable for manipulating cells in which the SSTRs are overexpressed, e.g. in certain tumors, and thus also for a well-calculated tumor therapy. Thus, a further preferred embodiment of the oligonucleotide conjugates according to the invention relates to those whose nucleic acid sequence is complementary to the nucleic acid sequence of a target molecule whose presence within the cell or whose over-expression is associated with the disease, e.g. a tumor. These target molecules comprise e.g. the mRNAs of oncogenes or proto-oncogenes, such as bcl-2, p53, N-myc, NF-KB, c-myc, Ha-ras, BCR-ALB, PKA(RIα) and c-raf-1.

The inhibition of bcl-2 is preferred, since a large number of tumor types shows an aberrant expression of the proto-oncogene bcl-2, which results in an increase of the concentration of the protein bcl-2. As is known, this protein inhibit apoptosis. Thus, the inhibition of the expression of the bcl-2 gene so as to effect apoptosis of the tumor cells and thus the elimination of the tumor, is a significant approach for a cancer therapy. Therefore, another preferred embodiment of the oligonucleotide relates to a molecule whose nucleic acid sequence is partially complementary to the nucleic acid coding for the proto-oncogene bcl-2. In a particularly preferred embodiment, the oligonucleotide molecule according to the invention comprises the nucleic acid sequence 5'-GTT CTC CCA GCG TGT GCC AT-3'.

Finally, the present invention relates to a medicament which contains an oligonucleotide conjugate according to the invention, optionally in combination with a pharmaceutically acceptable carrier. A person skilled in the art is familiar with suitable carriers and the formulation of such medicaments. The suitable carriers comprise e.g. phosphate-buffered common salt solutions, water, emulsions, e.g. oil/water emulsions, wetting agents, sterile solutions, etc. The medicament according to the invention may be present in the form of an injection solution, tablet, ointment, suspension, emulsion, suppository, etc. It may also be administered in the form of depots (microcapsules, zinc salts, liposomes, etc.). The kind of administration of the medicament depends *inter alia* on the form in which the active substance is present, it can be effected orally or parenterally. The methods for the parenteral administration comprise the topical, intra-arterial (e.g. directly into a carcinoma), intramuscular, intramedullary, intrathekal, intraventricular, intravenous, intraperitoneal, transdermal or transmucosal (nasal, vaginal, rectal, sublingual) administration. The administration can also be effected by microinjection. The suitable dosage is determined by the attending physician and depends on various factors, e.g. the age, sex, patient's weight, kind and stage of the disease, kind of administration, etc.

The oligonucleotide conjugate according to the invention is preferably used for antisense therapy, e.g. for treating viral diseases, inflammatory processes, asthmatic diseases, diseases of the CNS, cardio-vascular diseases or for cancer therapy, e.g. for treating small cell lung tumors, breast tumors and brain tumors.

### Description of the figures:

- Figure 1:: Diagram for the production of the Tyr³ octreotate ODN conjugates
- Figure 2:: Diagram for the production of the maleimido-modified Tyr³ octreotate
(a) step-wise elongation; (b) TI(TFA)₃; (c) Mal(CH₂)₅COOH, HOBTU, DIPEA; (d) TFA, H₂O, phenol, TIS.
- Figure 3:: Chemical structure of the Tyr³ octreotate ODN hybrid molecule
- Figure 4:: Chromatogramm of reverse phase HPLC
Conjugation of a thiol-modified oligonucleotide with the maleimido peptide ***4***. (A) reaction mixture after four hours; I: shortened sequences, II: ODN dimer by-product; III: conjugate; IV: excess maleimido peptide. (B) : purified conjugate ***6***
- Figure 5:: Melting curves of modified and non-modified 20-mer ODNs in 50 mM Tris/HCl (pH 7.5), 0.15 M NaCl.
Of complementary phosphorodiester strands having antisense bcl-2 phosphorothioate (O), sense bcl-2 phosphorothioate (•), ODN conjugate ***5*** (□), ODN conjugate ***6*** (■), and control strand antisense bcl-2 phosphorodiester (◆). For a better overview only every tenth measuring point is shown.
- Figure 6:: Displacement of ¹²⁵I-Tyr³ octreotide of rat cortex membranes
The measurements were determined from three experiments. The ordinate represents the specific binding in percent. It corresponds to the total binding minus binding in the presence of the ODN conjugates ***5*** (□); solid line), ***6*** (O; dotted line) and ***7*** (◆; broken line)

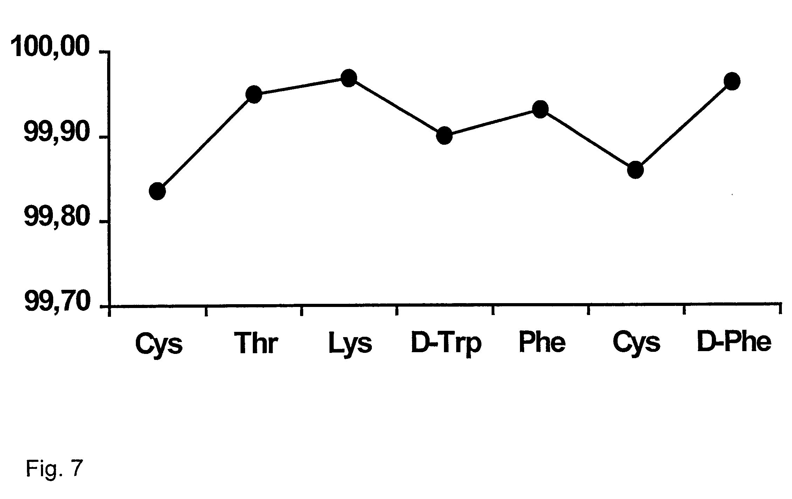

- Figure 7:: Acetylation yields of the couplings in the peptide portion (the values were obtained by assessing the quantitative ninhydrine test)
- Figure 8:: Synthesis diagram for the composition of the PNA peptide conjugate
(a) step-wise extension (HATU); (b) TI(TFA)₃; (c) step-wise extension (HATU); (d) TFMSA cleavage
- Figure 9:: Synthesis diagram for charging the resin
In order to minimize the charging degree, the Fmoc-protected amino acid derivative (Fmoc-AS) was used in stoichiometric amounts
- Figure 10:: Organ distribution of the ¹²⁵I-labeled PNA conjugate with and without blocking (by co-injection of an excess of octreotide), as compared to the organ distribution of ¹²⁵I-3-Tyr octreotide
- Figure 11:: Chemical structure of the octreotate-PNA conjugate
The sequence of the base is: H-AGC GTG CGC CAT CCC-OH

Abbreviations used: Acm: acetamidomethyl; Boc: tert.-butyloxycarbonyl; DCM: dichloromethane; DIPEA: diisopropylethylamine; DMF: dimethylformamide; DMSO: dimethylsulfoxide; DTT: dithiotreitol; FCS: fetal calf serum; Fmoc: 9-fluorenylmethyl methoxycarbonyl; For: formyl; HATU: O-(7-azabenzotriazole-1-yl)-1,1,3,3-tetramethyluronium hexafluorophosphate; HOBTU: 2-(lH-benzotriazole-1-yl)-1,1,3,3-tetramethyluronium hexafluorophosphate; MALDI: matrix assisted laser desorption ionization; MBHA: 4-methylbenzylhydrylamine; ODN: oligodeoxyribonucleotide; PAM: 4-(oxymethyl)phenylacetamidomethyl; RP-HPLC: reverse phase high performance liquid chromatography; SPPS: solid phase peptide synthesis; SSTR: somatostatin receptor; TEAA: triethylammoniumacetate; TFA: trifluoroacetic acid; TFMSA: trifluoromethane sulfonic acid; TIS: triisopropylsilane; Z: benzyloxycarbonyl.

The following examples explain the invention.

### Example 1: General methods

The peptides were analyzed and separated by means of HPLC on a "Gynotech P-580" system (Gynotech, Germering, Germany) which was equipped with a variable "SPD 6-A UV detector" and a "C-R5A integrator" (both devices from Shimadzu, Duisburg, Germany). The columns used were "Nucleosil C₁₈" 5 µm, 250 x 4 mm (Machery & Nagel, Düren, Germany), LiChrosorb RP-select B 5 µm, 250 x 4 mm and "LiChrosorb RP-select B" 10 µm, 250 x 10 mm (Merck, Darmstadt, Germany). The ODNs were purified on a "Waters" HPLC system on "PRP-1 Material" 7 µm, 305 x 7 mm (Hamilton, Bondauz, Switzerland). The UV measurements and the melting point studies were carried out with a "Varian Cary 13" spectrometer for U.V. light connected to an interface using a computer (company Varian, Palo Alto, U.S.A.). mass spectrometric analyses of the peptides and the oligonucleotides were carried out with a MALDI instrument ("Maldi-1" (= matrix-supported laser desorption/ionization; company Kratos Instruments, Chestnut Ridge, New York, U.S.A.). ¹H and ¹³C NMR spectrums of maleimido peptide **4** were recorded on an "AM 250" spectrometer (company Bruker Analytik GmbH, Rheinstetten, Germany) and are expressed as δ units relative to the CD₃OD (δ = 49.3 for ¹³C). The peptides were synthesized manually in an SPPS reactor or manually. The oligonucleotides were produced on a "Model 394" DNA synthesis device (Applied Biosystems, Foster City, U.S.A.) or on a "PerSeptive Expedite 8900" synthesis device (PerSeptive Biosystems, Hamburg, Germany). Lyophilization was carried out on an "α1-2" lyophilizer (Christ, Osterode, Germany). Membrane-binding studies were carried out on a filtration device produced especially for this purpose. The animals were kept and treated in accordance with the German law governing the protection of animals (authorization 35-9185.81/66/99, district presidency Karlsruhe).

All standard reagents were obtained from Merck (Darmstadt, Germany). The chemicals for the peptide synthesis were bought from Novabiochem (Läufelingen, Switzerland), N-maleinimido-6-capronic acid, thallium(III)trifluoroacetate and TIS from Fluka (Buchs, Switzerland). The chemicals for the oligonucleotide synthesis were provided by Perkin-Elmer-Applied Biosystems (Norwalk, Connecticut) or PerSeptive Biosystems (Hamburg, Germany). The PNA monomers and HATU were bought from PerSeptive Biosystems (Hamburg, Germany). The radioisotope Na¹²⁵I was procured from Amersham Pharmacia Biotech (Freiburg, Germany). The culture medium F12 (HAM) which contains L-glutamine, and penicillin streptomycin and fungizone/amphotericin were supplied by Gibco (Renfrewshire, U.K.). The reagent EDITH for the sulfurization was also supplied by PerSeptive Biosystems. The thiol linker phosphoroamidite was obtained from Glen Research (Sterling, Minnesota, U.S.A.). Anhydrous solvents were bought from Merck, PerSeptive Biosystems and SDS (Peypin, France). Tyr³ octreotide was produced by means of SPPS. ¹²⁵I-Tyr³ octreotide was produced by iodizing Tyr³ octreotide using the chloramine-T method according to Bakker et al., J. Nucl. Med. 9 (1990), 1501-1509. The resulting product was purified over HPLC and stored for further use at -80°C. The radioisotope Na¹²⁵I was obtained from Amersham Pharmacia Biotech (Freiburg, Germany). The complementary unmodified phosphodiester ODNs and phosphorothioate ODNs for the melting temperature analysis 5'-GTT CTC CCA GCG TGT GCC AT-3' (antisense) and 5'-ATG GCA CAC GCT GGG AGA AC-3' (sense) were synthesized according to standard methods.

### Example 2: Synthesis of the maleimido-modified Tyr³ octreotate

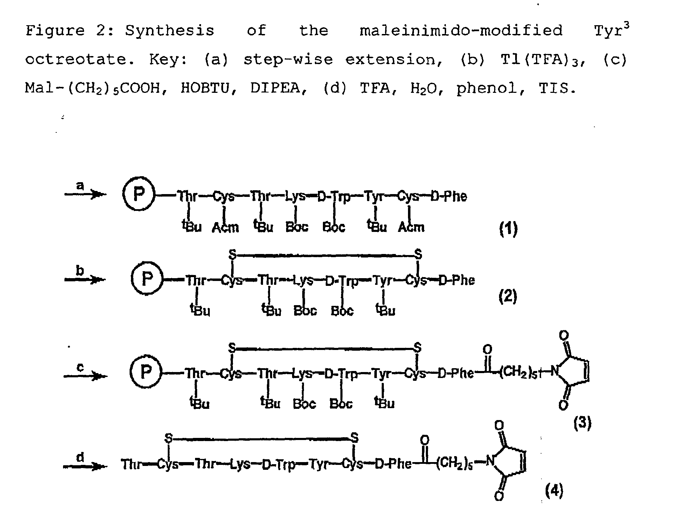

Although octreotate only contains eight amino acid residues, the synthesis is rendered more difficult by various structural features, in particular cyclization of the disulfide bridges cannot be harmonized with the introduction of amino-reactive and thiol-reactive groups, required for the conjugation. Therefore, a synthesis protocol was developed in the present invention which permits the solid-phase formation of the disulfide bridges and the subsequent linkage with the reactive group. In this connection, the peptides were joined by means of Fmoc chemistry to 1 g "Fmoc-Thr(^{t}B)-Wang" resin (0.61 mmol/g) (company of Novabiochem, Läufelingen, Switzerland) (figure 2). With this procedure the tyrosine residue was also introduced to enable radioactive labeling with iodine. The conjugation of the maleimido portion to the peptide prevented the subsequent cyclization of the disulfide. N^{α}-Fmoc amino acids with the following side chain protective groups were used: Cys(Ac-m), Lys(Boc), Thr(^{t}Bu), D-Trp(Boc) and Tyr(^{t}Bu). All coupling steps were carried out in DMF. The peptide chains were constructed manually in accordance with a modified *in situ* neutralization cycle (Schnölzer et al., Int. J. Peptide Protein Res. 40 (1992), 180-193). This cycle consisted of double decoupling ( 1 min. and 5 min.) with 50 % piperidine in DMF and 10 min. coupling with 4 equivalents Fmoc amino acid (0.4 M in DMF, 5 min. incubation with 3.9 equivalents HOBTU and 6 equivalents DIPEA). After conclusion of the reaction the resin (1.75 dry weight) was treated with piperidine in DMF so as to remove the protecting group from the terminal α-amino group of the peptide. An aliquot was cleaved and analyzed over HPLC, product ***1*** forming with a yield of over 90 %. 200 mg of the resin-bound peptide ***1*** were cyclized with two times the molar excess of thallium(III)trifluoroacetate in DMF at room temperature. It could be shown by analysis of a small aliquot that the formation of product ***2*** was substantially concluded within one hour. After thorough washing, N-maleinimido-6-capronic acid was coupled, as described above, the resin was washed and dried overnight in a vacuum. The cleavage was carried out with 5 ml 37:1:1:1 TFA/H₂O/phenol/TIS at room temperature for two hours. The resin was filtered off and washed. The peptide was precipitated by gradual addition of tert.-butyl-methylether at 4°C. The purification was made using reverse phase HPLC on the "RP-selectB" column with the following gradient: 20 % B -> 50 % b in 7.5 min. and 50 % B -> 100 % B in 5 min. (A = H₂O, B = acetonitrile, both with 0.1 % TFA); flow rate: 4 ml/min. Under these conditions the peptide eluted after 10.2 min. Following the lyophilization, 44 mg product ***4*** were obtained as a loose powder (43.6 % total yield). The purified peptide was characterized by means of mass spectrometry. For C₅₉H₇₅N₁₁O₁₅S₂ [M + H]⁺ m/z 1243.4 were calculated; found value: 1244.2. ¹³C NMR (CD₃OD) δ = 20.02 (q), 20.58 (q), 22.89 (t), 26.28 (t), 27.14 (t), 27.70 (2 C) (t), 29.24 (t), 31.42 (t), 36.75 (t), 38.4 (t), 39.34 (t), 40.19 (t), 40.65 (t), 46.45 (t), 46.52 (t), 53.86 (d), 54.16 (d), 54.43 (d), 55.18 (d), 56.42 (d), 57.68 (d), 59.68 (d), 60.55 (d), 68.55 (d), 69.09 (d), 110.41 (s), 112.33 (d), 116.23 (2 C), 119.53 (d), 120.05 (d), 122.57 (d), 124.83 (d), 127,74 (d), 128.66 (s), 128.91 (s), 129.34 (2 C) (d); 130.66 (2 C) (d), 131.54 (2 C) (d) , 125.32 (2 C) (d), 137.99 (s), 138.56 (s), 157.35 (s), 171.26 (s), 172.11 (s), 172.60 (2 C) (s), 172.74 (s), 173.26 (s), 173.57 (s), 174.20 (s), 174.35 (s), 175.00 (s), 175.36 (s).

After the oxidation, the absorption of the ß-carbon atoms of the cysteine moves considerably to greater δ values in the ¹³C-NMR spectrum (e.g. from δ = 25.5 to δ = 38.9 for glutathione). Thus, this chemical shift can be used for determining the oxidation status of the peptides with disulfide bridges. The ¹³C NMR signals of the two C_{ß} of the cysteine in compound ***4*** appeared at 46.4 and 46.5 ppm, which indicates the formation of a disulfide bridge.

### Example 3: Synthesis and purification of the 5' thiol ODNs

The ODNs exclusively containing phosphorothioate compounds 5'-GTT CTC CCA GCG TGT GCC AT-3' (antisense), 5'-ATG GCA CAC GCT GGG AGA AC-3' (sense) and 5'-TAC CGT GTG CGA CCC TCT TG-3' (non-sense) were synthesized by means of the ß-cyanoethyl-phosphoroamidite chemistry in the 1 µm range. The acetylation was carried out by means of 0.1 M acetic anhydride/tetrahydrofuran (THF) and 0.1 M imidazole/THF. The sulfurization was made by means of the EDITH reagents (3-ethoxy-1,2,4-dithiazoline-5-one). The commercially available thiol linker phosphoroamidite having a spacer of six carbon atoms (1-O-dimethoxytrityl-hexyl-disulfide-1'-[(2-cyano-ethyl)-(N,N-diisopropyl)]phosphoroamidite (Glen Research) was coupled to the 5' end. An acetonitrile wash step followed the last coupling. The resin was dried in an argon stream and treated at 55°C with concentrated ammonia for 12 hours, which contained 0.1 M DTT, the removal of the protecting groups of the thiol protection and the separation from the resin also taking place simultaneously (Gottschling et al., Bioconjugate Chem. 9 (1998), 831-837). The resin was removed by filtration and washed with concentrated ammonia. After rotation evaporation of the resulting solution a clear residue was left which was dissolved in sterile water. In order to remove excess DTT, the solution was passed over an "NAP-10" gel filtration column. The ODN-containing fractions were immediately used for conjugation with the peptide.

### Example 4: Synthesis of the Tyr³ octreotate oligodeoxyribonucleotide conjugate

The 5'-thiol ODNs were added to the maleimido peptide ***4*** (five times the excess) in aqueous 0.1 M TEAA, pH 6.5, which contained 20 % DMF. The pH value was adjusted by adding 1 M TEAA, pH 6.5. The mixture was incubated at room temperature for 4 hours, and it turned out that this time was sufficient for a complete conjugation according to an analytical HPLC. For purifying the conjugates over RP-HPLC the following buffers were used: buffer A: 5 % acetonitrile in 0.1 M triethylammonium acetate, pH 6.5; buffer B: 70 % acetonitrile in 0.1 M triethylammonium acetate, pH 6.5; a linear gradient of 0 % - 100 % B (2 ml/min.) was used over a period of 30 min. The conjugates eluting after 22.3 min. were collected and lyophilized. The yields were between 34 and 42 % (based on the amount of the starting 5'-thiol ODNs). The conjugates were characterized by means of "MALDI-TOF" analysis. ***5*:** m/z = 7822.9 [M + H]⁺ (C₂₅₈H₃₃₅N₇₉O₁₂₀P₂₀S₂₃: calculated 7819.96 g/mol); ***6*:** m/z = 7936.8 [M + H]⁺ (C₂₆₀H₃₃₁N₉₅O₁₁₂P₂₀S₂₃: calculated 7936.07 g/mol); and ***7*:** m/z = 7822.9 [M + H]⁺ C₂₅₈H₃₃₅N₇₉O₁₂₀P₂₀S₂₃: calculated 7819.96 g/mol).

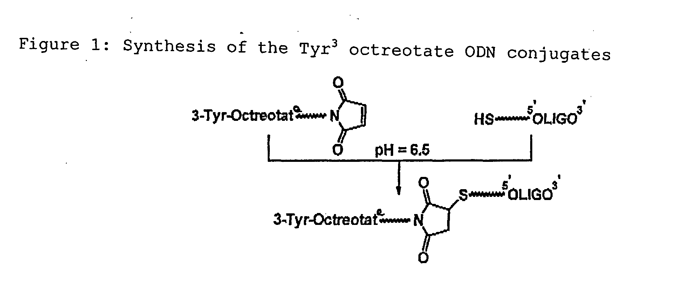

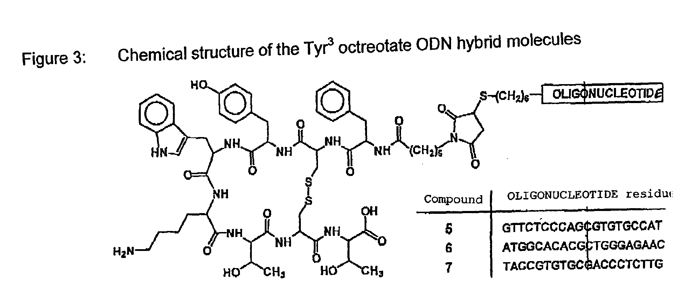

The production of the Tyr³-octreotate oligodeoxyribonucleotide conjugate with a stable thioether bond is also shown by way of diagram in figure 1 and the chemical structure of the individual Tyr³ octreotate oligodeoxyribonucleotide conjugates is illustrated in figure 3.

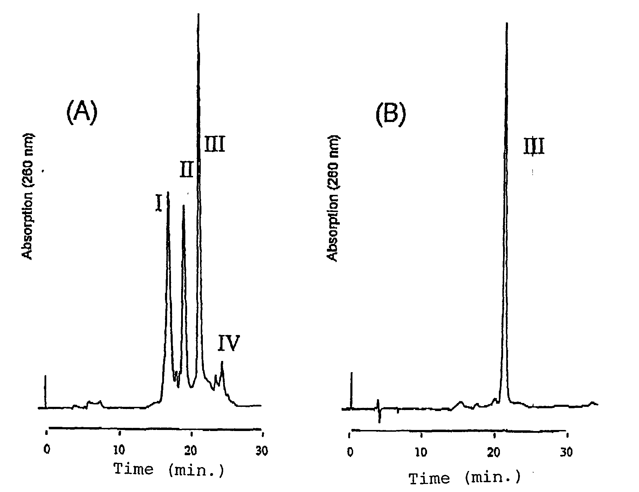

The chromatogram of the mixture of the crude products shows four peaks at about 16.8, 19.2, 21.1 and 24.8 min., which correspond to shortened sequences, the ODN dimer by-product, the conjugate and excess maleimido peptide (figure 4). Due to the considerable differences existing as regards the size and polarity the conjugates could easily be separated from the contaminants and excess educt ***4***.

### Example 5: Melting temperature analysis of the hybridization

The melting temperature analyses were used for determining the influence of the peptide portion on the hybridization efficiency of the antisense ODN peptide conjugates to the complementary strand. The dissociation of duplexes was determined which had been formed from the equimolar concentrations of the ODN conjugates and an unmodified 20-mer ODN target molecule. The measurements were carried out three times in each case in closed quartz cuvettes (1 cm path length) at 260 nm. The samples were produced as solutions of the two complementary oligomers in 1000 µl buffer with 0.5 OD. 50 mM Tris/HCl, pH 7.5, which contained 0.15 M NaCl, was used as the buffer. The melting curves were measured with a temperature gradient of 30 to 90°C with a heating and/or cooling rate of 0.5°C/min. The hybridization of the oligonucleotides was effected before the analysis in all samples by five minutes of heating to 90°C and subsequent slow cooling to room temperature. The analysis was carried out by means of the "Varian thermal" software (company Varian, Palo Alto, U.S.A.). All Tₘ values were calculated from the first derivative of the melting curve and they represent average values obtained from various analyses (+ standard deviation). The maximum insecurity as to the Tₘ data, based on repeated experiments, is approximately + 0.5°C.

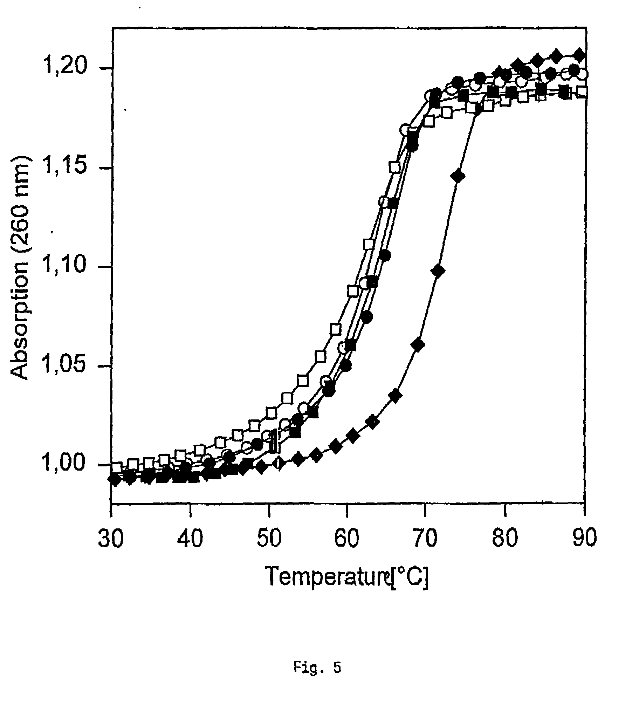

The Tₘ of the unmodified 20-mer starting phosphodiester sequence was 73.1 + 0.2°C. Compared therewith Tₘ values of conjugates ***5*** (65.0 + 0.0°C) and ***6*** (63.8 + 0.5°C) were relatively low. It is known that the Tₘ of a phosphorothioate drops by about 0.5°C/nucleotide as compared to the corresponding phosphodiester ODN (Freier, in: Antisense Research and Applications. Editor: Crooke, S.T., Lebeu, B., CRC Press Boca Raton, FL (1993), 67-82). Thus, the Tₘ curves were typical of those obtained with unmodified ODNs. In order to confirm this, unmodified phosphorothioate ODNs were produced. The resulting Tₘ values of the antisense strand (64.8 + 0.5°C) and the sense strand (66.2 + 0.5°C confirmed the correctness of the prediction. In figure 5, the Tₘ curves of the duplexes which contained conjugates ***5, 6*** and the unmodified phosphorothioate ODNs, were compared with the unmodified duplex. These results refer to the fact that the peptide portion at the 5' terminus of the 20-mer ODN does not impair the hybridization efficiency.

### Example 6: SSTR binding assays

For accurately determining the competitive displacement reaction, the concentration of the conjugates was determined by means of the millimolar absorption coefficient. In this connection, it was assumed that εₘ is the ODN εₘ and the peptide εₘ: εₘ = Σ ((nA x 15.4 + nC x 7.3 + nG x 11.7 + nT x 8.8) x 0.9) + (nTrp x 5.0 + nTyr x 1.4 + nPhe x 0.2). Using this equation the following εₘ values were determined: ***5*** = 180.5; ***6*** = 212.7; and ***7*** = 180.5. For the binding assays rat cortex membranes were resuspended at a protein concentration of 500 µg/ml in incubation buffer (10 mM HEPES, pH 7.6 with 5 % BSA fraction V, MgCl₂ (10 mM) and bacitracin (20 µg/ml)). 100 µg protein were used per assay. The cell membranes (200 µl) were mixed with 30 µl incubation buffer with increasing concentrations of the competitor (conjugates **5-7)** (10⁻⁵ to 10⁻¹⁰ mol/l). About 20.000 cpm ¹²⁵I-Tyr³ octreotide (about 20 pM) in 70 µl incubation buffer were added. After one hour at room temperature, the incubation was terminated by rapid filtration over "GF/B" glass fiber filters (Whatman, Springfield Mill, U.S.A.) which had been moistened with 1 % BSA-containing buffer. The filters were washed with ice-cold buffer (10 mM Tris, 150 mM NaCl) and the bound radioactivity was determined by means of a gamma counter. The non-specific binding measured by measuring the binding in the presence of excess non-labeled octreotide (10⁻⁶ mol/l) was about 10 - 20 % of the total binding. The specific binding was defined as total binding minus non-specific binding. The results are indicated as values of the specific binding determined from three experiments.

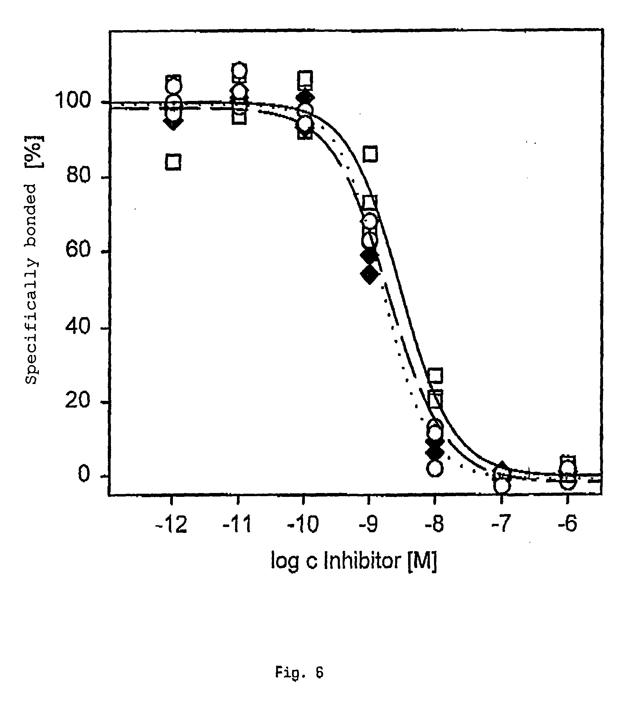

Figure 6 shows the progressive displacement of ¹²⁵I-Tyr³ octreotide of rat cortex membranes. The three examined conjugates bonded with high affinity in the lower nanomolar range, the IC50 values of conjugates ***5, 6*** and ***7*** were 1.83 + 0.17 nM, 2.52 + 0.43 nM and 1.88 + 0.47 nM, respectively. The similar affinities clearly show that the sequence of the ODN does not influence markedly the receptor affinity.

### Example 7: Synthesis and organ distribution study of PNA peptide conjugates

As explained in more detail below, PNA peptide conjugates were synthesized by means of Boc chemistry. These conjugates were modified with tyrosine at the N terminus and then labeled radioactively with ¹²⁵I by the chloramine T method. In organ distribution studies, the uptake of these synthetic oligonucleotide conjugates into the SSTR-positive CA20948 pancreas tumors in Lewis rats was examined. A selective uptake, which could be inhibited with an excess of octreotide, into the tumor tissue could be found here.

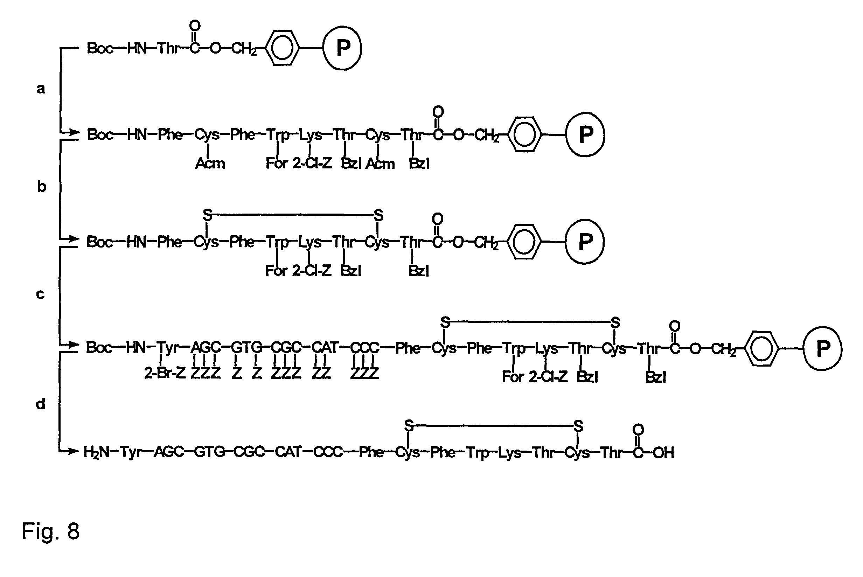

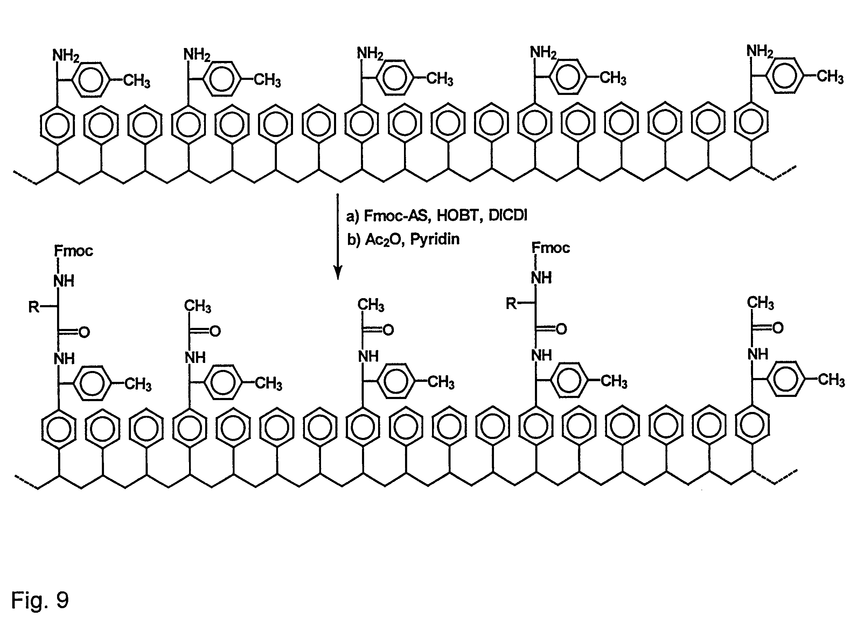

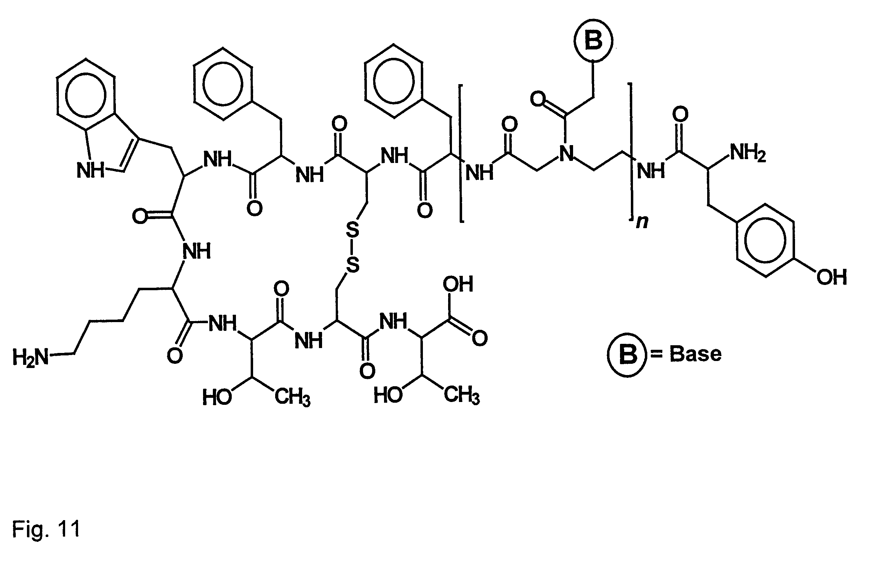

### Structure of a PNA peptide conjugate (figures 8, 9, 11) :

### Design:

The peptide H-D-Phe-Cys-Phe-D-Trp-Lys-Thr-Cys-Thr-OH (octreotate) was coupled with a PNA sequence which is complementary to the nucleotides -3 to +12 (region of the AUG start codon) of the Bcl-2 sequence. The sequence used is: H-AGC GTG CGC CAT CCC-OH. Of this sequence a specific inhibition of the Bcl-2 protein synthesis was described (Mologni et al., Biochem. Biophys. Res. Commun. 264, pages 537-543 (1999)).

### Resin:

In order to avoid aggregation of the growing oligomers by association between various chains, the charging degree of the resin had to be reduced. For this purpose, substoichiometric amounts of an Fmoc-protected amino acid were used for the first coupling reaction. Capping was carried out with Ac₂O:pyridine in DMF. The Fmoc protecting group of the first amino acid was used to facilitate the quantification of the charging degree.

### Peptides:

The peptide portion was built up at the PAM resin. The amino acids were protected with N-Boc. The protecting groups of the side chains were: Cys(Acm), Lys(2-Cl-Z), Thr(Bzl) D-Trp(For). The resin was swelled overnight in DCM and washed with DMF. A reaction cycle consisted of: a) cleavage of the N-terminal Boc group by a short wash step, followed by shaking the resin with TFA/p-cresol (95.5, v/v) for 1 min., b) pre-activation of a solution of 4 eq. of the Boc-protected monomer for 2 min. in DMSO with 3.9 eq. HATU and 10 eq. DIEA, c) washing the resin with DMF (1 min. vacuum-supported flow), d) coupling of the pre-activated amino acid for 3 min., e) washing of the resin with DMF (1 min. vacuum-supported flow). After each coupling step, a sample was taken to determine the acylation efficiency. It was determined by means of the quantitative ninhydrin reaction (Sarin et al., Anal. Biochem. 117, pages 147-157 (1981)). A sample was separated from the resin, deprotected and analyzed by means of HPLC. This analysis resulted in the fact that the H-D-Phe-Cys(Acm)-Phe-D-Trp-Lys-Thr-Cys(Acm)-Thr-OH had formed in a yield of > 95 %. The resin-bound peptide was cyclized with two times the excess of T1(FFA)₃ in DMF at room temperature. Analysis of deprotected samples separated from the resin led to the result that the formation of H-D-Phe-cyclo[Cys-Phe-D-Trp-Lys-Thr-Cys]-Thr-OH was substantially concluded within one hour.

### PNA:

Using N-Boc-protected PNA monomers, the PNA oligomers were built up on PAM or MBHA resin. The resin was swelled in DCM overnight and washed with DMF. The FMOC protecting group of the first amino acid was cleaved by treatment with 50 % piperidine in DMF for 2 x 5 minutes. The monomers were dissolved in 1:1 DMF/pyridine (A, C, T monomer) or 1:1 DMSO/pyridine (G monomer). A reaction cycle consisted of: a) cleavage of the N-terminal Boc group by shaking the resin with TFA/p-cresol (95:5, v/v) for 2 x 2 min. b) pre-activation of a solution of 5 eq. of the Boc-protected PNA monomer with 4.9 eq. HATU and 10 eq. DIEA for 2 min. (final concentration 0.1 M), c) washing of the resin with DMF/DCM (1:1, v/v) followed by pyridine, d) coupling of the pre-activated amino acid for 15 min., e) washing of the resin with DMF/DCM (1:1, v/v), f) shaking with Ac₂O:pyridine:DMF (1:10:10, v/v/v) for 5 min., g) washing with DMF/DCM (1:1, v/v), h) shaking with DMF/piperidine (95:5 v/v), i) washing with DMF/DCM (1:1, v/v). In place of a repeated ninhydrin test, small samples were removed after every fourth coupling. As described below, these samples were deprotected and analyzed by means of HPLC and MALDI-TOF-MS. The deprotection of the 2-Cl-Z protecting group was rather slow and therefore a reaction time of 2 hours was necessary to terminate the cleavage. The coupling rates of the PNA monomers decreased with increasing length of the oligomer. Hence it was found that a double coupling from the 10th to 15th PNA monomer was necessary. The couplings with tyrosine were carried out in substantially the same way as described for the couplings of the peptide portion. Prior to the TFMSA cleavage, the N-terminal Boc group was cleaved with TFA/-p-cresol (95:5, v/v) and the resin was washed with DCM and dried in a nitrogen atmosphere. The complete PNA peptide conjugate was cleaved with TFA/TFMSA/p-cresol (40:40:10, v/v/v) at room temperature for 2 hours and deprotected. The resin was filtered and washed with TFA. The peptide was precipitated by the addition of tert.-butyl methylether at 4°C.

### Purification:

The purification was carried out by means of HPLC (RP-selectB column 10 mm internal diameter) with a gradient of 20 % B -> 50 % B in 7.5 min. and 50 -> 100 % B in 5 min. (A = H₂O and B = acetonitrile, both with 0.1 % TFA), flow rate = 4 ml/min. Under these conditions, the product eluted after 10.2 min. Following lyophilization, 44 mg of the conjugate were obtained as a flaky white composition. The identity of the purified product was confirmed by MALDI-MS.

### Iodination:

The iodination was carried out with the chloramine-T method. Na¹²⁵I (specific activity 0.6 TBq/mg, with a concentration of 3.7 GBq/mL) was mixed with the conjugate in phosphate buffer, pH 7.5, which contained 20 % DMF. The chloramine-T solution was prepared with a concentration of 2 mg/mL in phosphate buffer, pH 7.5. Chloramine-T was added and the reaction mixture was quenched after 30 seconds at room temperature with a saturated solution of methionine in phosphate buffer (pH 7.5). The entire reaction mixture was purified by means of RP-HPLC. In this connection, the iodinated product was obtained in a yield of about 50 %.

### Organ distribution studies:

Organ distribution studies of the conjugate were carried out with tumor-bearing rats. CA 20948-tumor-bearing Lewis rats were used as animal model. The CA 20948-tumor cells express stably somatostatin receptors. The tumor cells are thawed, washed with PBS and suspended carefully. The cell suspension was transplanted subcutaneously into the flank of male Lewis rats (220-250 g) (about 2 x 10⁶ cells in 0.1 ml). Within two weeks, palpable tumors having a diameter of 0.25 - 1 cm developed in the CA 20948 tumor model. The animals were given an injection of about 1 MBq of the radioactively labeled somatostatin derivative into the caudal vein. After 1 hour, the animals were killed and dissected. Various organs/tissue samples (tumor, blood, heart, lung, liver, stomach, kidney, spleen, muscle) were removed, weighed and the activity in the gamma counter was determined. In this way, % ID/g values could be obtained. The competitive uptake could be examined by injection of 100 µg octreotide, 30 min. prior to the application of the radioactive octreotide derivative.

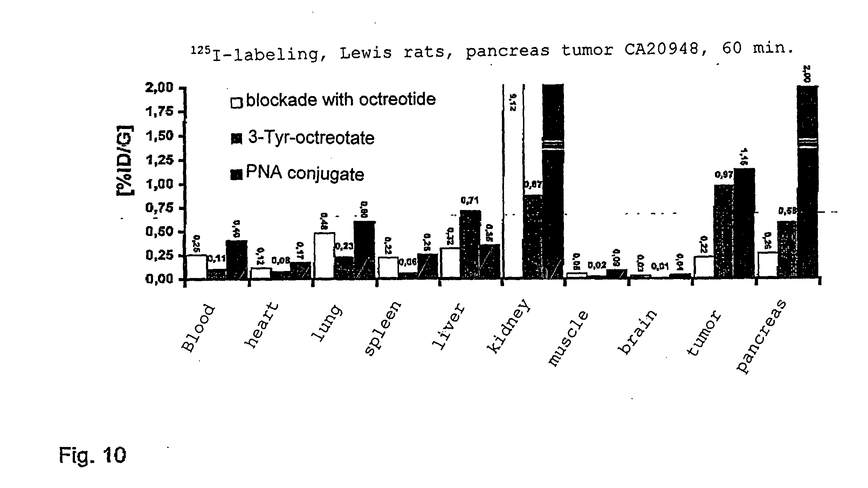

A comparison of the organ distribution with 3-Tyr octreotide showed that the conjugate has approximately the same concentration in tumor and pancreas (also an SSTR-positive tissue) (figure 10).

**Table:**

| Organ distribution of the ¹²⁵I-labeled PNA peptide conjugate | | | |
|---|---|---|---|
| The indicated values are % ID/g values | | | |

| | PNA conjugate | blockade with octreotide | 3-Tyr-octreotate |
|---|---|---|---|
| blood | 0.40 | 0.25 | 0.11 |
| heart | 0.17 | 0.12 | 0.08 |
| lung | 0.60 | 0.48 | 0.23 |
| spleen | 0.25 | 0.22 | 0.06 |
| liver | 0.35 | 0.32 | 0.71 |
| kidney | 13.65 | 9.12 | 0.87 |
| muscle | 0.09 | 0.05 | 0.02 |
| brain | 0.04 | 0.03 | 0.01 |
| tumor | 1.15 | 0.22 | 0.97 |
| pancreas | 2.00 | 0.26 | 0.58 |

## Claims

1. An oligonucleotide conjugate comprising: (a) an oligonucleotide at least part of whose sequence is complementary to an intracellular nucleic acid sequence; and (b) a somatostatin analog.

2. The oligonucleotide conjugate according to claim 1, wherein the oligonucleotide is an oligodeoxyribonucleotide.

3. The oligonucleotide conjugate according to claim 1 or 2, wherein the phosphodiester compounds in the oligonucleotide are partially or fully replaced by phosphorothioate compounds.

4. The oligonucleotide conjugate according to claim 1, 2 or 3, wherein the 3' end in the oligonucleotide is covalently bonded to a propanediol group.

5. The oligonucleotide conjugate according to any one of claims 1 to 4, wherein the somatostatin analog is octreotide or octreotate or a derivative thereof.

6. The oligonucleotide conjugate according to any one of claims 1 to 5, wherein the somatostatin analog is covalently bonded to the 5' end of the oligonucleotide molecule.

7. The oligonucleotide conjugate according to any one of claims 1 to 6, wherein the somatostatin analog is covalently bonded to a base present in the oligonucleotide molecule via a spacer.

8. The oligonucleotide conjugate according to claim 5 or 6, wherein the somatostatin derivative is Tyr³ octreotate.

9. The oligonucleotide conjugate according to any one of claims 1 to 8, wherein the intracellular nucleic acid sequence is an mRNA or viral RNA.

10. The oligonucleotide conjugate according to claim 9, wherein the intracellular nucleic acid sequence is the coding portion of an mRNA.

11. The oligonucleotide conjugate according to any one of claims 1 to 10, wherein the oligonucleotide has a length of 8 to 50 nucleotides.

12. The oligonucleotide conjugate according to claim 11, wherein the oligonucleotide has a length of 12 to 20 nucleotides.

13. The oligonucleotide conjugate according to any one of claims 1 to 12, wherein the oligonucleotide is partially complementary to the nucleic acid coding for the proto-oncogene bc1-2.

14. The oligonucleotide conjugate according to claim 13, which comprises the nucleic acid sequence 5'-GTT CTC CCA GCG TGT GCC AT-3'.

15. The oligonucleotide conjugate according to any one of claims 1 to 13, wherein the oligonucleotide is a peptide-nucleic acid derivative (PNA).

16. A pharmaceutical preparation, containing the oligonucleotide conjugate according to any one of claims 1 to 15, optionally in combination with a pharmaceutically compatible carrier.

17. Use of the oligonucleotide conjugate defined according to any one of claims 1 to 15, for the antisense therapy.

18. Use according to claim 17 for cancer treatment, for treating viral diseases, for treating inflammatory processes, for treating asthmatic diseases, for the therapy of diseases of the central nervous system and for the therapy of cardiovascular diseases.
